# EUROPEAN PATENT APPLICATION

(11) **EP 1 762 201 A1**
(43) Date of publication of application: **14.03.2007**
(21) Application number: 06254544.7
(22) Date of filing: 31.08.2006
(51) Int. Cl.: A61F 2/40

(54) **Method and apparatus for a glenoid prosthesis**

(30) Priority: 08.09.2005 US 222308
(71) Applicant: Biomet Manufacturing Corporation, Warsaw, IN 46581 (US)
(72) Inventor: Berelsman, Brian K., Warsaw, Indiana 46580 (US); Winslow, Nathan A., Warsaw, Indiana 46582 (US); Shultz, Jason M., Hamilton, Ohio 45013 (US)
(74) Representative: Giles, Ashley Simon

(57) **Abstract**

A method and apparatus for a glenoid prosthesis. According to various embodiments, the glenoid prosthesis may include a first member defining a cavity including a wall, and a second member defining a projection operable to interconnect with the cavity of the first member. Alternatively, the glenoid prosthesis may include a first member including at least one projection and at least one anchor, the at least one projection and at least one anchor operable to engage the anatomy or the glenoid prosthesis may include a first member including at least one resiliently deformable projection and a second member defining an aperture operable to interconnect with the at least one resiliently deformable projection of the first member to couple at least one of the first member or second member to the anatomy.

## Description

### FIELD OF THE INVENTION

The present invention relates generally to biomedical implants, and particularly to a method and apparatus for a glenoid prosthesis.

### BACKGROUND OF THE INVENTION

Many portions of the human anatomy naturally articulate relative to one another. Generally, the articulation between the portions of the anatomy are substantially smooth and without abrasion. This articulation is allowed by the presence of natural tissues, such as Cartilage and strong bone.

Over time, however, due to injury, stress, degenerative health issues and various other issues, articulation of the various portions of the anatomy may become rough or impractical. For example, injury may cause the cartilage or the boney structure to become weak, damaged, or non-existent. Therefore, the articulation of the anatomical portions is no longer possible for the individual. At such times, it may be desirable to replace the anatomical portions with a prosthetic portion such that normal or easy articulation may be reproduced.

A humerus generally articulates within a glenoid surface or cavity in a shoulder. After injury or other degenerative processes, the glenoid surface may become rough or damaged. Therefore, it may be desirable to replace the glenoid surface with a prosthetic.

### SUMMARY OF THE INVENTION

A modular prosthesis for replacing a portion of the anatomy including a first member defining a cavity, and the cavity includes a wall. The modular prosthesis further includes a second member defining a projection operable to interconnect with the cavity of the first member. In addition, at least one of the wall or the projection is resiliently deformable and at least one of the first member or second member is fixed to the anatomy.

A modular prosthesis for replacing a portion of the anatomy is provided. The modular prosthesis includes a first member including at least one projection and at least one anchor. The at least one projection is operable to engage the anatomy and is comprised of a metallic material. The at least one anchor is fixedly coupled to the anatomy.

A modular prosthesis for replacing a portion of the anatomy is provided. The modular prosthesis includes a first member including at least one resiliently deformable projection and a second member including an aperture operable to interconnect with the at least one resiliently deformable projection of the first member. In addition, at least one of the first member or second member of the modular prosthesis is fixed to the anatomy.

A method for replacing a portion of the anatomy is provided, including drilling at least one hole into the anatomy. Next, a first member is provided and the first member includes a cavity. A second member is also provided which is operable to engage the anatomy. Then the second member is inserted into the anatomy to engage the anatomy and next, the cavity of the first member is interconnected with the second member to couple the first member to the anatomy.

Further areas of applicability of the present invention will become apparent from the detailed description provided hereinafter. It should be understood that the detailed description and specific examples, while indicating the preferred embodiment of the invention, are intended for purposes of illustration only and are not intended to limit the scope of the invention.

### BRIEF DESCRIPTION OF THE DRAWINGS

The present invention will become more fully understood from the detailed description and the accompanying drawings, wherein:

Figure 1A is a side view of a glenoid prosthesis according to various embodiments;

Figure 1B is a perspective view of the glenoid prosthesis of Figure 1A;

Figure 1C is an alternate perspective view of the glenoid prosthesis of Figure 1A;

Figure 1D is an side view of an alternate glenoid prosthesis of Figure 1A;

Figure 1E is a perspective view of the glenoid prosthesis of Figure 1D;

Figure 1F is a coupling member configured for use with the glenoid prosthesis of Figure 1A;

Figure 1G is an alternate coupling member configured for use with the glenoid prosthesis of Figure 1A;

Figure 2 is a detailed perspective view of the glenoid prosthesis of Figure 1A including the coupling member shown in Figure 1F;

Figure 3A is an environmental view of a first procedure employed to prepare a selected portion of the anatomy for the glenoid prosthesis according to various embodiments;

Figure 3B is an environmental view of a second procedure employed to prepare a selected portion of the anatomy for the glenoid prosthesis according to various embodiments;

Figure 3C is an environmental view of the glenoid prosthesis of Figure 1A prior to insertion into the anatomy;

Figure 3D is an environmental view of the glenoid prosthesis of Figure 1A after it has been inserted into the anatomy;

Figure 3E is a cross sectional view of the glenoid prosthesis of Figure 3C along lines 3E-3E;

Figure 3F is a cross sectional view of the glenoid prosthesis of Figure 3D along line 3F-3F;

Figure 4A is a side view of an alternate glenoid prosthesis according to various embodiments;

Figure 48 is a perspective view of the alternate glenoid prosthesis of Figure 4A;

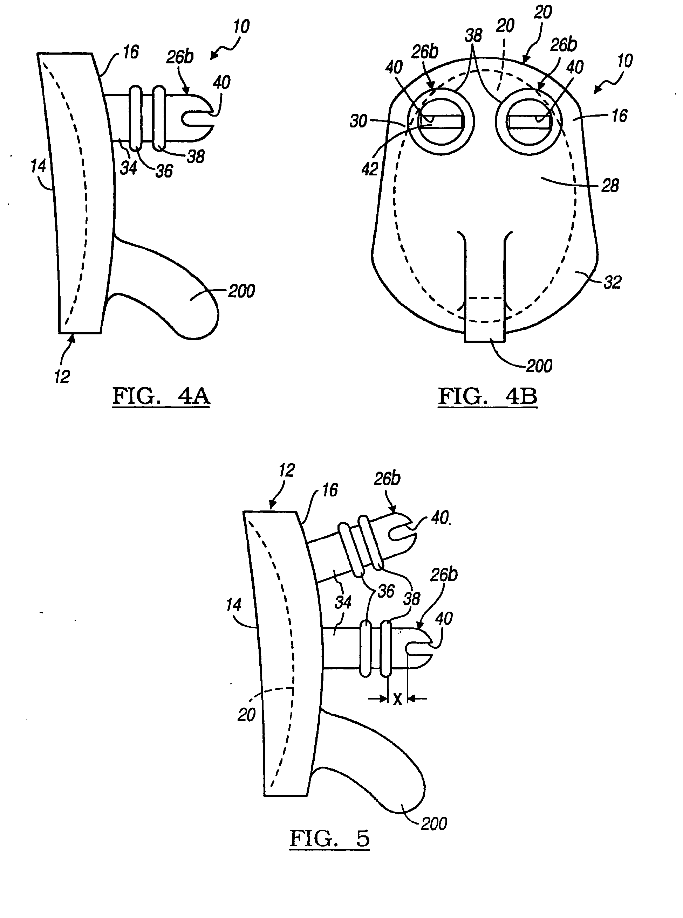

Figure 5 is an side view of an alternate glenoid prosthesis of Figure 4A;

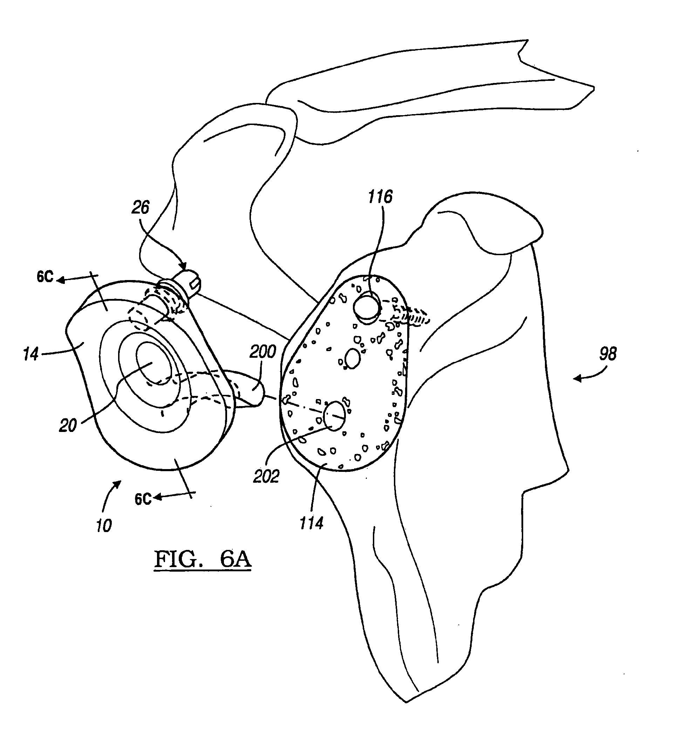

Figure 6A is an environmental view of the glenoid prosthesis of Figure 4A prior to insertion into the anatomy;

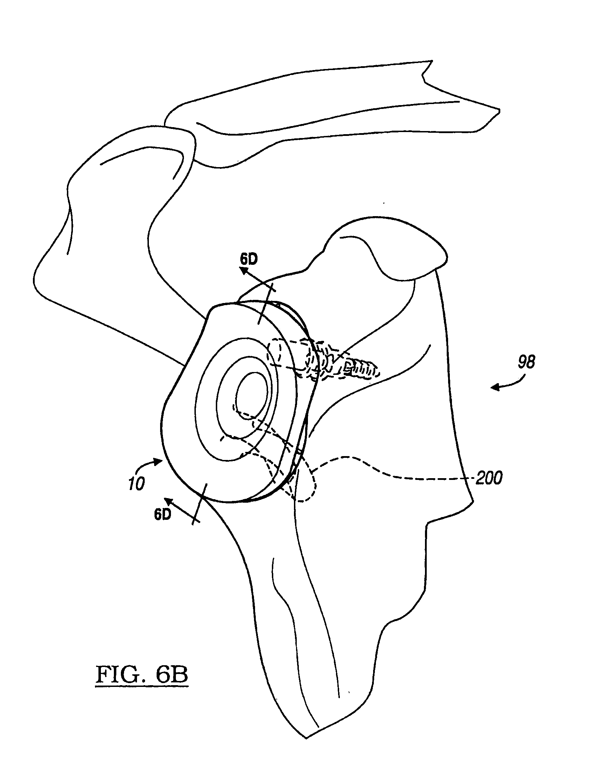

Figure 6B is an environmental view of the glenoid prosthesis of Figure 4A after it has been inserted into the anatomy;

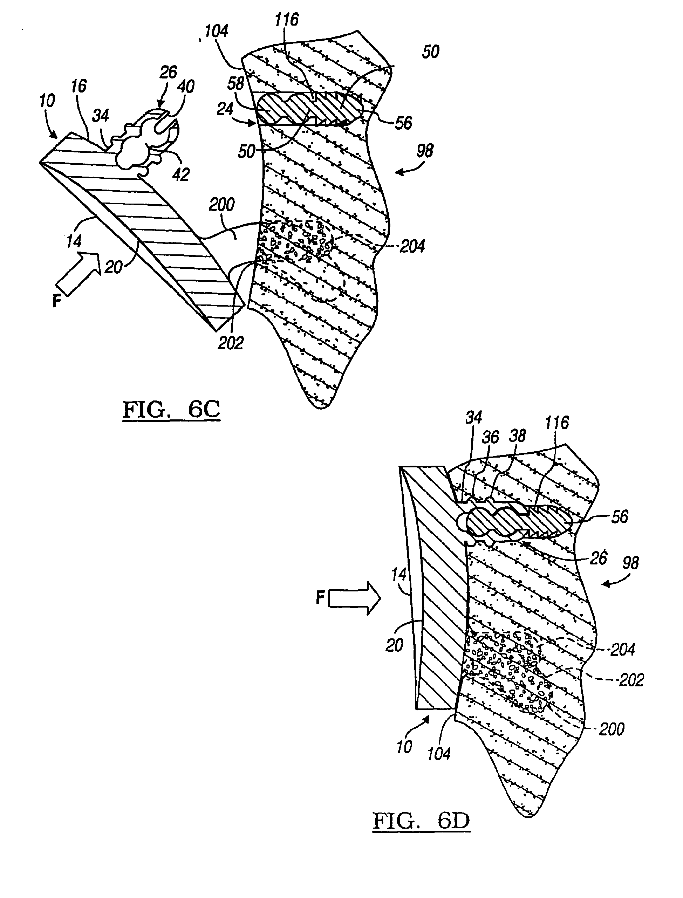

Figure 6C is an cross sectional environmental view of the glenoid prosthesis of Figure 6A along line 6C-6C of Figure 6B;

Figure 6D is a cross sectional environmental view of the glenoid prosthesis of Figure 6B along line 6D-6D of Figure 6B;

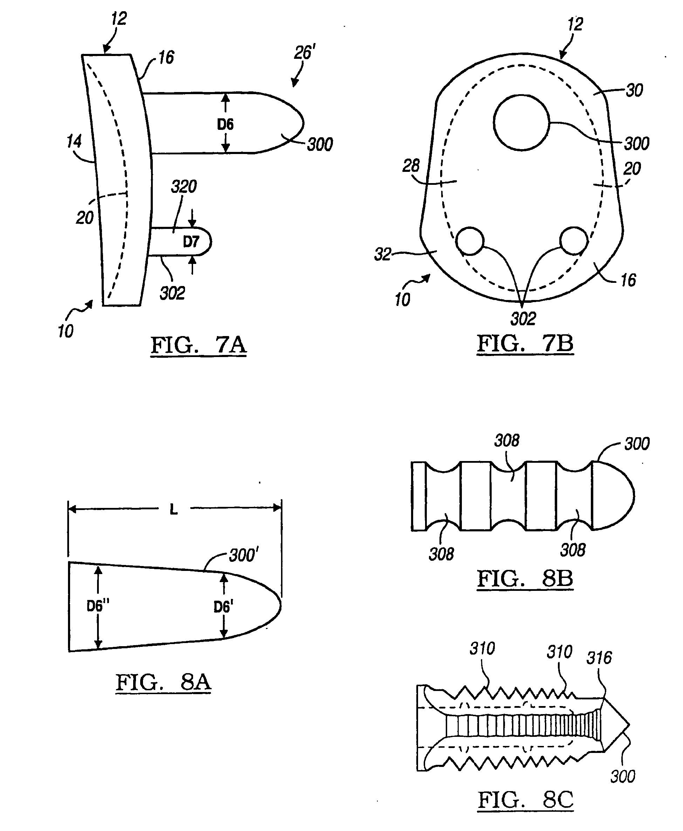

Figure 7A is a side view of a second alternate glenoid prosthesis according to various embodiments;

Figure 7B is a perspective view of the glenoid prosthesis of Figure 7A;

Figure 8A is a detailed perspective view of an alternate feature of the glenoid prosthesis of Figure 7A according to various embodiments;

Figure 8B is a detailed perspective view of a second alternate feature of the glenoid prosthesis of Figure 7A according tp various embodiments;

Figure 8C is a detailed perspective view of a third alternate feature of the glenoid prosthesis of Figure 7A according to various embodiments;

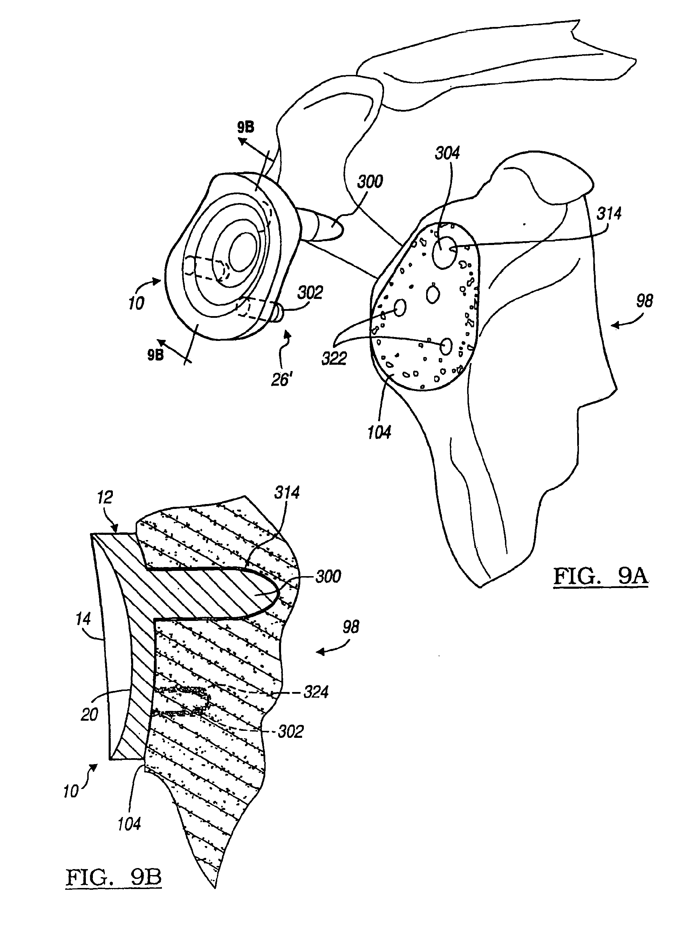

Figure 9A is an environmental view of the glenoid prosthesis of Figure 7A prior to insertion into the anatomy;

Figure 9B is a cross-sectional environmental view of the glenoid prosthesis of Figure 7A along line 9B-9B of Figure 9A, after it has been inserted into the anatomy;

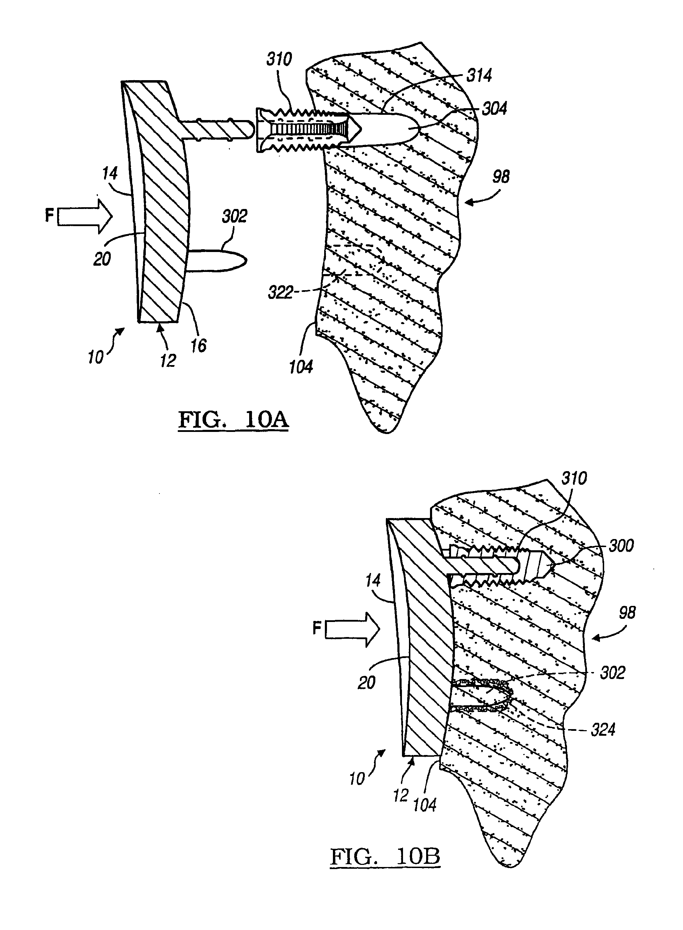

Figure 10A is a cross sectional environmental view of the glenoid prosthesis of Figure 7A along line 9B-9B of Figure 9A, including the third alternate feature of Figure 8C prior to being fully inserted into the anatomy;

Figure 10B is a cross sectional environmental view of the glenoid prosthesis of Figure 7A along line 9B-9B of Figure 9A, including the third alternate feature of Figure 8C after being fully inserted into the anatomy;

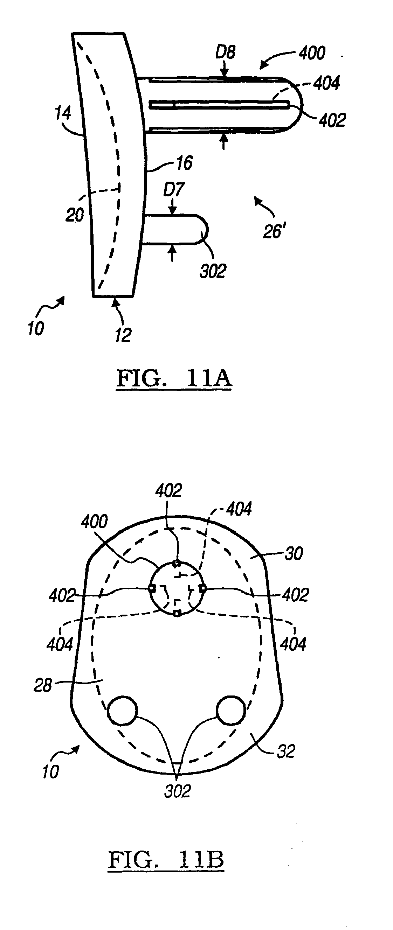

Figure 11A is a side view of the alternate glenoid prosthesis of Figure 7A incorporating a fourth alternate feature according to various embodiments;

Figure 11B is a perspective view of the glenoid prosthesis of Figure 11A;

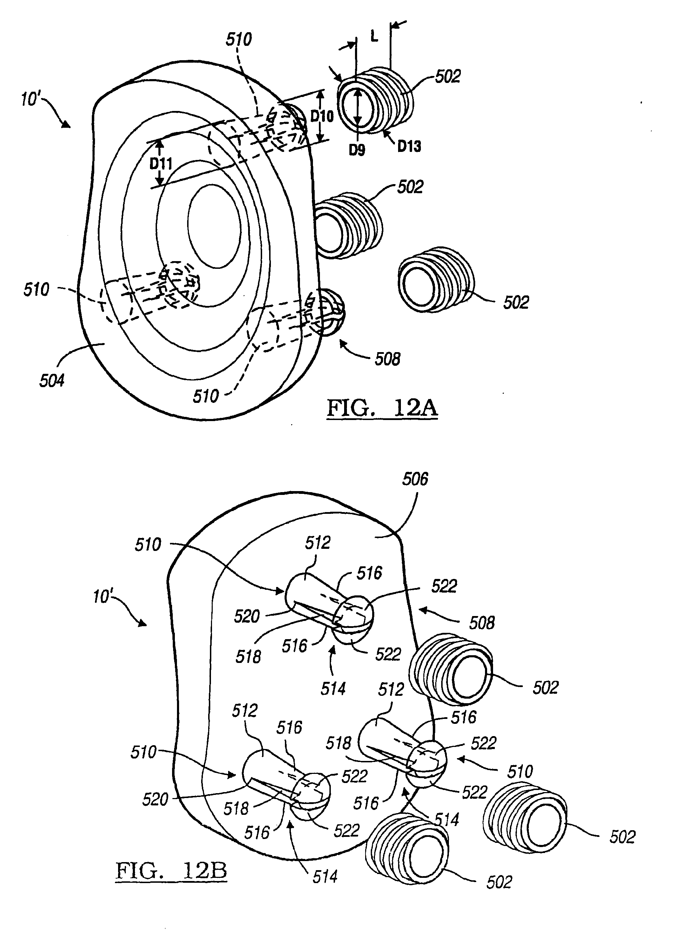

Figure 12A is a front perspective view of a second alternative glenoid prosthesis according to various embodiments;

Figure 12B is a rear, perspective view of a second alternative glenoid prosthesis according to various embodiments;

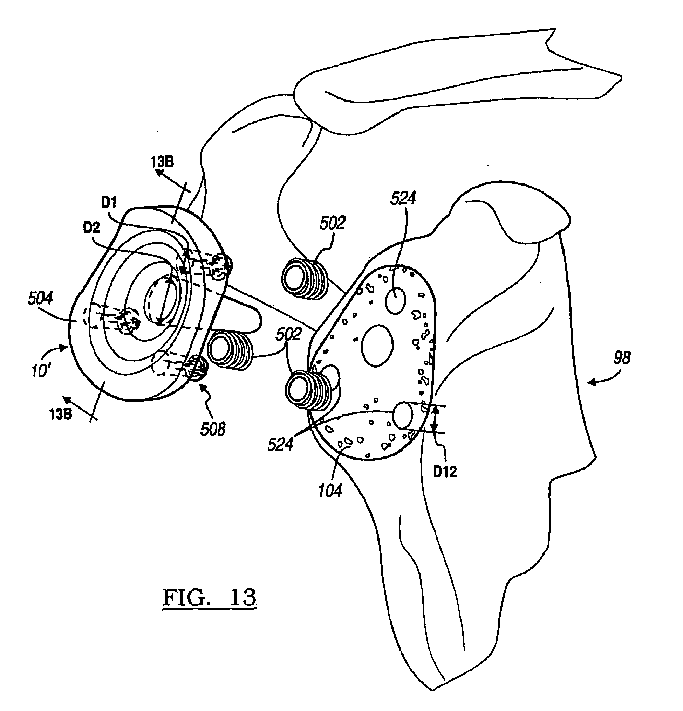

Figure 13 is an environmental view of the glenoid prosthesis of Figure 12A prior to insertion into the anatomy;

Figure 14A is a cross sectional environmental view of the glenoid prosthesis of Figure 12A along line 13B-13B of Figure 13, prior to being fully inserted into the anatomy; and

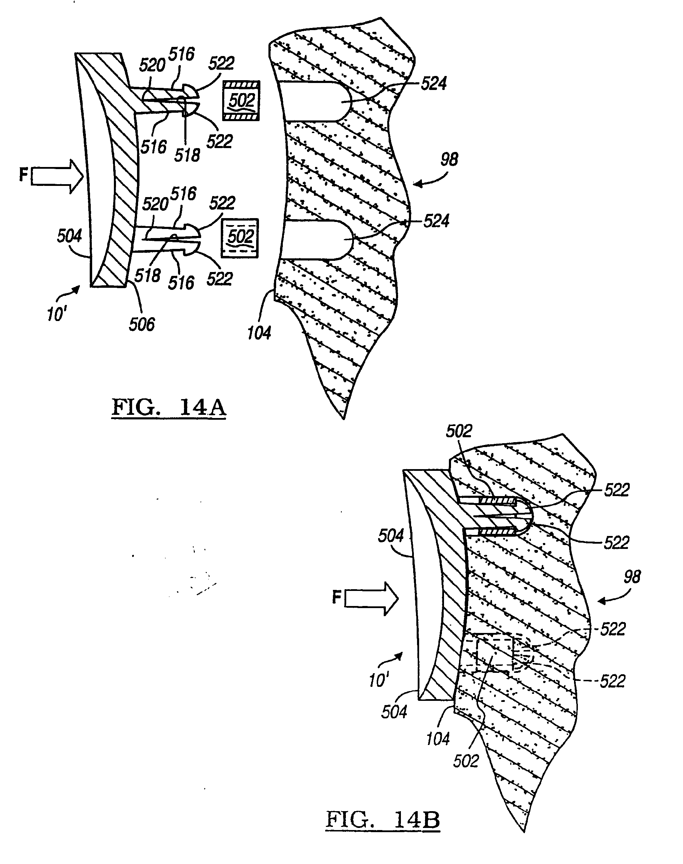

Figure 14B is a cross sectional environmental view of the glenoid prosthesis of Figure 12A along line 13B-13B of Figure 13, after being fully inserted into the anatomy.

### DETAILED DESCRIPTION OF VARIOUS EMBODIMENTS

The following description of various embodiments is merely exemplary in nature and is in no way intended to limit the invention, its application, or uses. Although the following description is related generally to a prosthesis that can be positioned in a prepared portion of the anatomy, such as a glenoid cavity in the shoulder, it will be understood that the prosthesis, as described and claimed herein, can be used with any appropriate surgical procedure. Therefore, it will be understood that the following discussions are not intended to limit the scope of the appended claims.

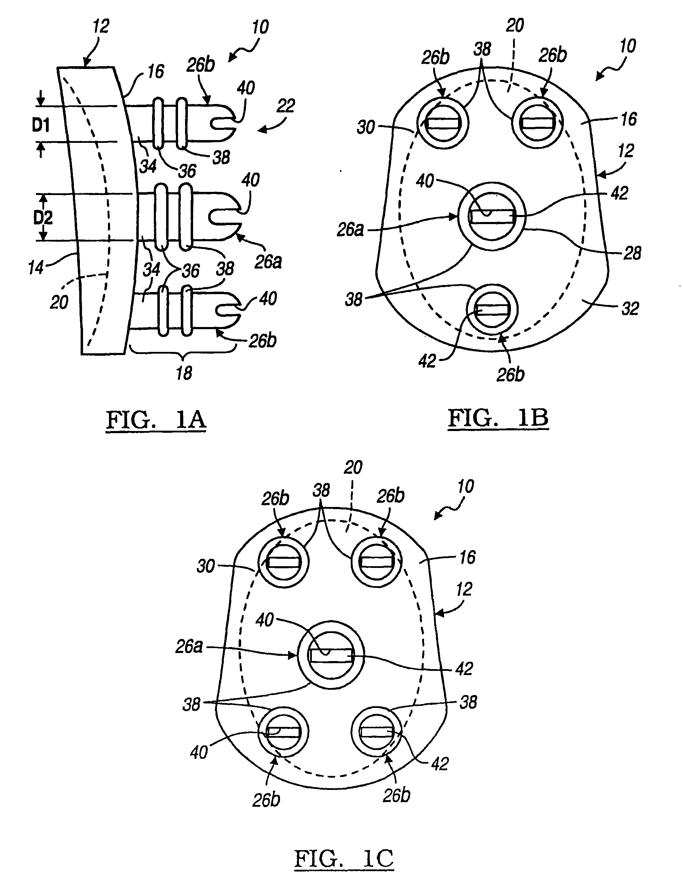

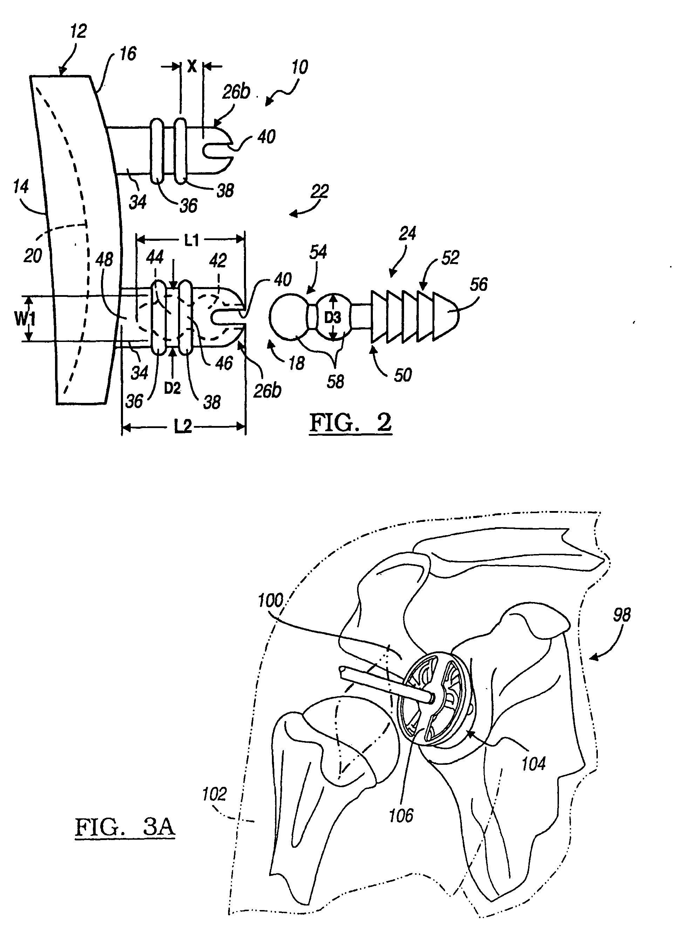

With reference to Figures 1A, 1B and 2, a glenoid prosthesis 10 is illustrated. The glenoid prosthesis 10 may include a body 12 generally shaped to provide a first bearing surface 14 configured to enable a humeral implant or a portion of the anatomy (not shown) to rotate against the first surface 14. The body 12 also generally includes a second surface 16 and an anatomy attachment system 18.

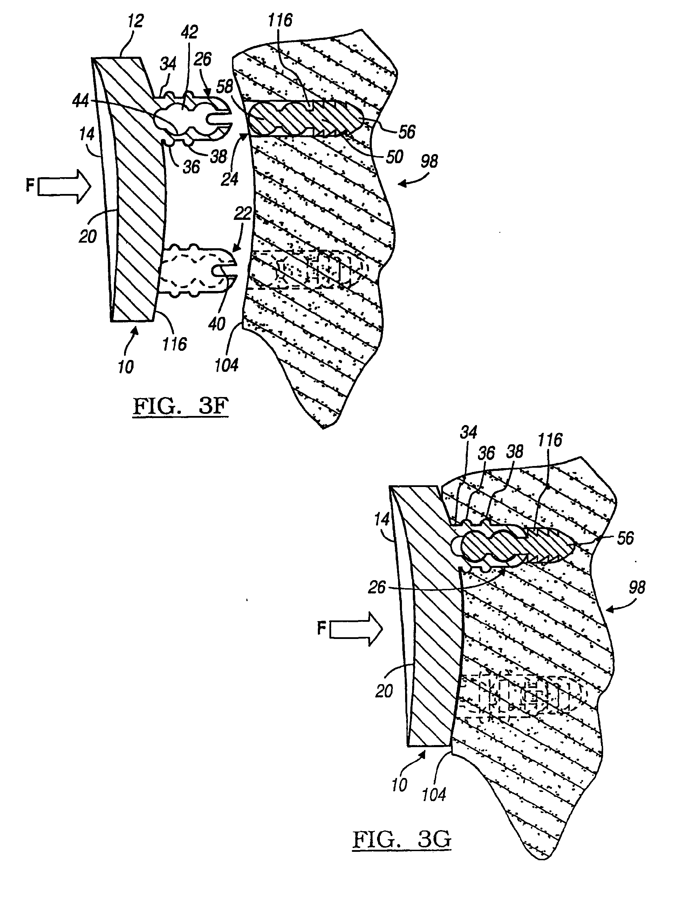

The first surface 14 of the glenoid prosthesis 10 includes a generally concave depression 20 configured for receipt and articulation of a humerus or humeral implant (not shown). It should be understood that although the first surface 14 is illustrated with the concave depression 20, the first surface 14 could be configured as required for any desired surgical replacement. The second surface 16 is generaiiy configured to conform to a surface on the mating anatomy such that once the glenoid prosthesis 10 is attached to the anatomy; the second surface 16 is adjacent to the surface on the anatomy (as best shown in Figure 3F). According to various embodiments, the second surface 16 may be generally curved. With reference back to Figure 1A, 1B and 2, the second surface 16 is coupled to the anatomy attachment system 18.

The anatomy attachment system 18 includes a peg system 22 and a fixation system 24. The peg system 22 is coupled to the second surface 16 of the glenoid prosthesis 10 and interconnects with the fixation system 24 to couple the glenoid prosthesis 10 to the anatomy. The peg system 22 includes at least one projection or peg 26, but generally includes more than one peg 26. The pegs 26 may be disposed in any desired configuration on the second surface 16 of the glenoid prosthesis 10, and the pegs 26 may be designed to each have different shapes and sizes, as will be discussed in greater detail below.

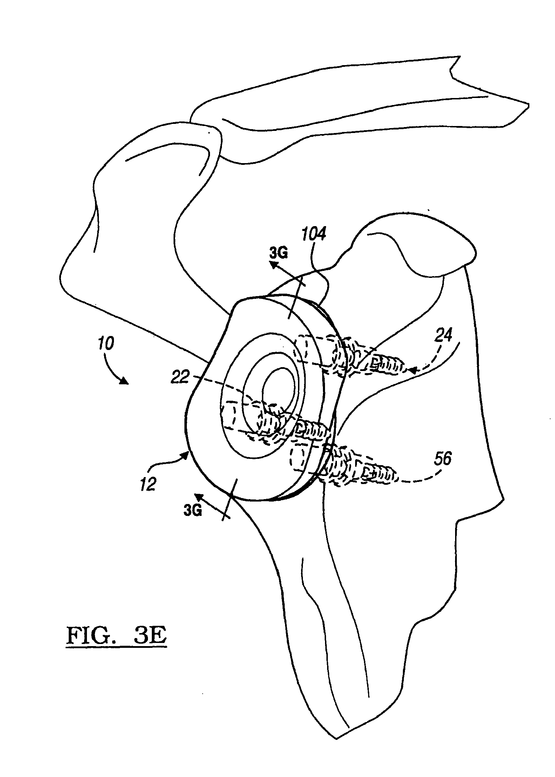

With reference to Figure 1A and 1B, the peg system 22 may include a plurality of pegs 26, which may include one primary peg 26a and three secondary pegs 26b. The primary peg 26a may be located in a central portion 28 of the glenoid prosthesis 10 to provide increased stability, while the three secondary pegs 26b may be spaced about the second surface 16 of the glenoid prosthesis 10. The primary and secondary pegs 26a, 26b act to secure a top portion 30 of the glenoid prosthesis 10 and a bottom portion 32 of the glenoid prosthesis 10 to the anatomy (as shown in Figure 3E) as will be discussed in greater detail below. Although Figure 1B illustrates the use of two secondary pegs 26b to secure the top portion 30 of the glenoid prosthesis 10 and one secondary peg 26b to secure the bottom portion 32 of the glenoid prosthesis 10, it will be understood that the bottom portion 32 could also be secured with two secondary pegs 26b and the top portion 30 second with one secondary peg 26b if desired.

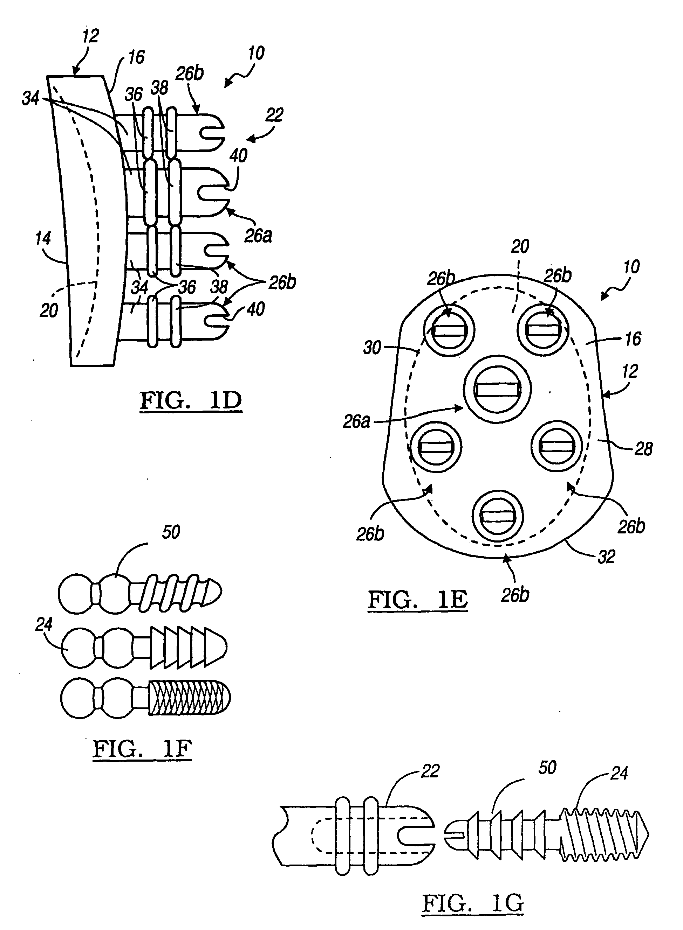

In addition, as best shown in Figure 1C, the top portion 30 and bottom portion 32 could be secured with two secondary pegs 26b, while the central portion 28 is secured with the primary peg 26a. This configuration may be desirable if adequate bone stock exists and additional fixation is deemed necessary. Alternatively, as shown in Figures 1D and 1E, the top portion 30 of the glenoid prosthesis 10 may be secured with two secondary pegs 26b, the central portion 28 of the glenoid prosthesis 10 can be secured with the primary peg 26a and two secondary pegs 26b, while the bottom portion 32 may be secured with one secondary peg 26b. The configuration shown in Figures 1D and 1 E may be desirable for situations requiring additional securement to the anatomy.

With reference to Figures 1A, 1B, 1C, 1D and 2, both the primary pegs 26a and secondary pegs 26b are generally configured similarly, with a diameter D1 of the secondary pegs 26b being smaller than a diameter D2 of the primary pegs 26a. The primary and secondary pegs 26a, 26b are generally comprised of UHMWPE, however, they could also be composed of polyethylene, polyether ether ketone or other suitable polymeric materials. Each of the primary pegs 26a and secondary pegs 26b are generally cylindrical and include a wall 34, a first annular flange 36, a second annular flange 38 and a slot 40. The wall 34 of each of the primary and secondary pegs 26a, 26b further defines a cavity 42.

The first annular flange 36 and second annular flange 38 operate to ensure that each of the primary and secondary pegs 26a, 26b fit securely with the anatomy as will be described in greater detail below. The first and second annular flanges 36, 38 are typically coupled to the exterior of the wall 34, and may be formed on the wall 34. Additionally, the first annular flange 36 and second annular flange 38 may be coated with a bio-compatible material, such as titanium plasma spray or cobalt chrome plasma spray, hydroxyapatite, calcium phosphate, or combinations thereof, to promote tissue growth. The first and second annular flanges 36, 38 are displaced a distance X from the slot 40.

The slot 40 may be configured to enable the fixation system 24 to couple the body 12 of the glenoid prosthesis 10 to the anatomy. The slot 40 is generally formed perpendicular to a centerline C of the primary and secondary pegs 26a, 26b, however, any other configuration would be permissible. Generally, the slot 40 enables the wall 34 to expand slightly during engagement with the fixation system 24, as Will be described in greater detail below. The slot 40 further provides entry to the cavity 42.

Each of the cavities 42 formed in the primary and secondary pegs 26a, 26b also provide the attachment point for connecting the body 12 of the glenoid prosthesis 10 to the anatomy. As best shown in Figure 2, the cavity 42 generally extends at least a length Li, approximately half a length L2 of the primary and secondary pegs 26a, 26b. The cavity 42 may include at least one curved portion 44, but typically includes two curved portions 44 separated by a ridge 46 and terminating in a smaller curved tip 48. The cavities 42 formed in each of the primary and secondary pegs 26a, 26b are generally approximately equally sized. The shape of the curved portions 44, ridge 46 and smaller curved tip 48 facilitate the engagement of the fixation system 24 with the cavity 42 to couple the peg system 22 to the fixation system 24.

With continuing reference to Figure 2, the fixation system 24 is shown to include at least one anchor pin 50. Generally, the number of anchor pins 50 may correspond one to one with the number of primary and secondary pegs 26a, 26b. The anchor pin 50 may be composed of a bio-compatible metallic material, such as titanium, however any suitable metallic material could be employed. The anchor pin 50 may include a bone fixation portion 52 and a coupler 54. The bone fixation portion 52 may include a barbed portion 56 or may be a pointed shaft (not shown) or threaded tip or any other mechanism capable of mechanically fastening the anchor pin 50 in a hole formed in the anatomy (as shown in Figure 3E).

As seen in Figures 1F and 1G, the coupler 54 may include at least one spherical protrusion 58, and typically may include two spherical protrusions 58. The spherical protrusions 58 are configured to fit within the cavity 42 of the primary and secondary pegs 26a, 26b. Generally, the spherical protrusions 58 are configured to facilitate a snap-fit with the cavity 42, as typically the spherical protrusions 58 are sized such that a diameter D3 of the spherical protrusion 58 is at least approximately the same size as a width W1 of the curved portion 44 of the cavity 42, although the diameter D3 of the spherical protrusion 58 may be greater than the width W1 of the curved portion 44 of the cavity 42 if a tighter fit is desired. Further, besides a snap-fit, other fastening mechanisms, such as expandable locking features (see Figure 1G), could be employed to fasten the anchor pin 50 to the primary and secondary pegs 26a, 26b.

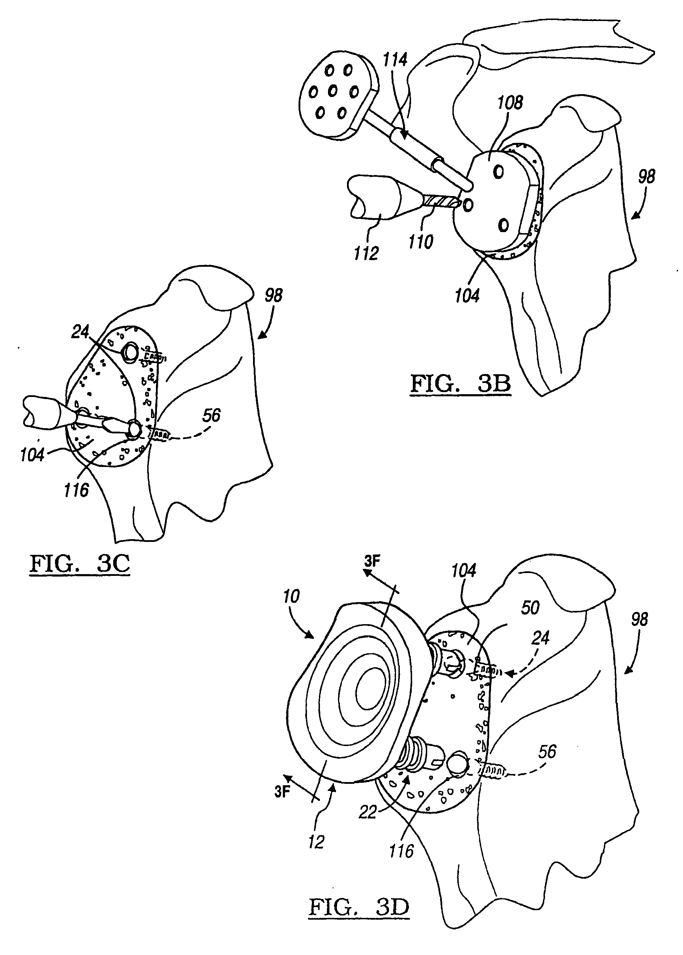

As best shown in Figures 3A, 3B and 3C, in order to secure the glenoid prosthesis 10 to the anatomy 98, a first incision 100 may be made into a selected portion of the skin 102 of a patient to provide access to the selected portion of the anatomy 98, in this case, the glenoid cavity 104. Next, the anatomy 98 may be reamed with a reamer 106 to provide a smooth interface for the glenoid prosthesis 10. Then, as shown in Figure 3B, the operator can position a guide block 108 over the glenoid cavity 104 to provide a locator for a drill bit 110 attached to a drill 112. An instrument 114 can be used to hold the guide block 108 on the glenoid cavity 104. Next, the drill bit 110 can be employed to create at least one hole 116 (shown in Figure 3C) in the glenoid cavity 104. The number of holes 116 depends upon the peg system 22 associated with the glenoid prosthesis 10. If the glenoid prosthesis 10 employs a peg system 22 including a primary peg 26a in combination with at least one secondary peg 26b, then the drill 112 may include a second bit (not shown) to enable the formation of different sized holes 116 in the glenoid cavity 104.

With continuing reference to Figure 3B and additional reference to Figures 3C, 3D and 3E, after the creation of the desired number of holes 116, the instrument 112 can be removed from the anatomy 98, allowing the guide block 108 to be removed from the glenoid cavity 104. Next, the appropriate number of anchor pins 50 can be inserted into the holes 116 in the glenoid cavity 104. Generally the number of anchor pins 50 will correspond at a one to one ratio with the number of holes 116 and primary and secondary pegs 26a, 26b in the peg system 22. After the anchor pins 50 are firmly secured in the holes 116, the primary and/or secondary pegs 26a, 26b of the peg system 22 can be coupled to the spherical projections 58 on the anchor pin 50 by applying a force F to the first surface 14 of the glenoid body 12. As the force F is applied to the first surface 14 of the glenoid prosthesis 10, the spherical protrusions 58 pass through the slot 40 and into the cavity 42 of each of the primary and/or secondary pegs 26a, 26b. Next, the spherical protrusions 58 each enter the corresponding curved portion 44 formed in the cavity 42.

It should be noted that the number of spherical protrusions 58 on the anchor pin 50 could be increased or decreased at a one to one ratio with curved portions 44 formed in the cavity 42 as necessary for the desired application. Once each of the spherical protrusions 58 engage their respective curved portions 44 of the cavity 42, the second surface 16 of the body 12 of the glenoid prosthesis 10 is generally adjacent to the glenoid cavity 104 as shown in Figures 3D and 3F. Thus, the peg system 22 and fixation system 24 act together to securely attach the glenoid prosthesis 10 to the anatomy 98.

Alternatively, as shown in Figures 4A, 4B and 5, the glenoid prosthesis 10 may include a keel 200 in addition to the peg system 22. As shown specifically in Figures 4A and 4B, the keel 200 may be employed on a glenoid prosthesis 10 including two secondary pegs 26b, or as illustrated in Figure 5, the keel 200 could be employed with a glenoid prosthesis 10 including three secondary pegs 26b, however, any possible combination of primary or secondary pegs 26a, 26b could be employed with the keel 200.

The keel 200 may be situated at any permissible location on the second surface 16 of the body 12, however, typically the keel 200 will be near the bottom portion 32 of the body 12 of the glenoid prosthesis 10. Additionally, the keel 200 could be formed integrally with the body 12 or could be coupled to the body 12 in a post processing step through any appropriate fastening method, such as screws, adhesives and the like. The keel 200 may be comprised of UHMWPE, however, the keel 200 could also be composed of polyethylene, polyether ether ketone or other suitable polymeric materials.

As illustrated in Figures 6A, 6B, 6C and 6D, in order to secure the glenoid prosthesis 10 including the keel 200 to the anatomy 98, after creating the incision 100 and preparing the glenoid cavity 104 with the reamer 106 as shown in Figures 4A and 4B, a guide block (not shown) may be placed onto the glenoid cavity 104 and may be held into place with the instrument 114 (not shown). Next, a drill may form the holes 116 for the insertion of the anchor pins 50 and an additional hole 202 for receipt of the keel 200 (not specifically shown). Typically, the length of the hole 202 is such that when the keel 200 is fully inserted into the hole 202, the glenoid prosthesis 10 abuts the glenoid cavity 104. In order to secure the keel 200 in the hole 202, generally a bone cement 204, such as polymethylmethacrylate bone cement, may be applied in the hole 202 prior to the insertion of the keel 200 in the hole 202. Typically the amount of bone cement 204 is such that when the keel 200 is fully inserted, the bone cement 204 fully covers the keel 200 without exiting the hole 202.

After the formation of the holes 116, 202 and the insertion of the bone cement 204 into the hole 202, as shown in Figure 6C, the anchor pins 50 can be secured into the holes 116 formed in the glenoid cavity 104. Next, as shown in Figure 6C, the keel 200 can be aligned with the hole 202, and as the force F is applied to the first surface 14 of the body 12 of the glenoid prosthesis 10, the keel 200 moves further into the hole 202 while the anchor pins 50 begin to enter the cavities 42 formed in the secondary pegs 26b. Once the anchor pins 50 are completely secured in the cavities 42 of the secondary pegs 26b and the glenoid prosthesis 10 abuts the glenoid cavity 104, the keel 200 will also be fully secured in the hole 202. The use of the keel 200 may be desirable to provide increased stability for the glenoid prosthesis 10 once it is attached to the glenoid cavity 104. Although the foregoing description described the use of secondary pegs 26b rather than primary pegs 26a, it will be understood that primary pegs 26a may also be employed either alone or in combination with the secondary pegs 26b.

According to various embodiments, as illustrated in Figures 7A and 7B, a glenoid prosthesis 10 may include an alternate peg system 22'. The alternate peg system 22' may include a primary peg 300 and at least one secondary peg 302. Generally, there may be two secondary pegs 302, however, any appropriate number of secondary pegs 302 could be used. The primary peg 300 may be coupled to the top portion 30 of the body 12 of the glenoid prosthesis 10 while the secondary pegs 302 may be coupled to the bottom portion 32 of the body 12, however, the primary peg 300 and secondary pegs 302 may be coupled to any desired location on the body 12.

The primary peg 300 may generally have a diameter D6, greater than a diameter D7 of the secondary peg 302. The primary peg 300 may be formed of a bio-compatible metallic material, such as, titanium, however other bio-compatible materials could be employed. In addition, the primary peg 300 may be coated with porous metal matrix, plasma, hydroxyapatite, calcium phosphate, or combinations thereof, to promote tissue growth.

The primary peg 300 may include various configurations to enable the primary peg 300 to be employed in a variety of surgical procedures. The primary peg 300 is adapted to fit into a corresponding hole 304 formed in the glenoid cavity 104 as will be discussed in greater detail below. As shown in Figure 8A, the primary peg 300 may have a first diameter D6' and a second diameter D6". The primary peg 300 may increase in diameter from diameter D6' to diameter D6", however, both diameter D6' and diameter D6" may still be of a diameter greater than the diameter D7 of the secondary peg 302, thus forming a primary peg 300' which is generally tapered along its length L. The tapered primary peg 300' may be employed for operations in which the size of the hole 304 may be desired to have a gradually reduced diameter within the glenoid cavity 104.

Alternatively, with reference now to Figure 8B, the primary peg 300 may also be configured with a plurality of grooves 308, however, it will be understood that the primary peg 300 may include a singular groove 308 (not shown). The use of grooves 308 may facilitate additional tissue and bone ingrowth on the primary peg 300. With reference to Figure 8C, the primary peg 300 may alternatively include at least one cutting tooth 310, which may be operable to further assist in engaging the primary peg 300 with the hole 304 formed in the glenoid cavity 104. Generally, however, the cutting tooth 310 can comprise a plurality of cutting teeth 310 which act to engage an inner surface 314 within the hole 304 in the glenoid cavity 104 as shown in Figures 10A and 10B. The cutting teeth 310 may be formed onto a surface 316 of the primary peg 300, or may be machined onto the primary peg 300 through any appropriate mechanical process, such as, broaching, forming, abrasion or other techniques.

With reference back to Figures 7A and 7B, the secondary pegs 302 generally have a surface 320 which may be uniform, however, the surface 320 of the secondary pegs 302 may include any appropriate feature as described in reference to the primary peg 300 such as cutting teeth 310, grooves 308 or a taper (not shown). Each of the secondary pegs 302 may be formed of a polymeric material, such as polyethylene, however any other suitable material could be used. The secondary pegs 302 are generally configured to be fixedly coupled into a plurality of corresponding holes 322 formed in the glenoid cavity 104, as shown in Figures 9A and 98. Typically, the secondary pegs 302 will be affixed into the corresponding holes 322 in the glenoid cavity 104 by an appropriate material 324, such as polymethylmethacrylate bone cement, biological fixation brought on by hydroxyapatite coatings, plasma spray coatings, etc. However, any other appropriate materials or mechanisms could be employed.

In order to attach the glenoid prosthesis 10 with the alternative peg system 22' to the glenoid cavity 104, the glenoid cavity 104 can be prepared as discussed previously, after forming the incision 100, by reaming the surface of the glenoid cavity 104 if necessary (as shown in Figures 3A and 3B). Next, the appropriate number of holes in the glenoid cavity can be formed using the guide block 108, the instrument 114 and a drill 112, with the guide block 108 including a larger opening for receipt of a drill bit to form the hole 304 for the primary peg 300 (not specifically shown). The guide block 108 may also include apertures to be used with the drill 112 and second drill bit to form the appropriate number of holes 322 in the glenoid cavity 104 for receipt of the secondary pegs 302 (not specifically shown).

After the holes 304, 322 have been formed, the material 324 may be inserted into the holes 322 in the glenoid cavity 104. Then, the glenoid prosthesis 10 can be aligned with the corresponding holes 304, 322 in the glenoid cavity 104 and a force F can be applied to the first surface 14 of the body 12 of the glenoid prosthesis 10 to enable the primary peg 300 and the secondary pegs 302 to begin entering the corresponding holes 304, 322 in the glenoid cavity 104. Depending upon the configuration of the primary peg 300, the primary peg 300 will generally rest within the hole 304 of the glenoid cavity 104, however, if the primary peg 300 is configured with at least one cutting tooth 310 as shown in Figures 10A and 10B, the primary peg 300 will engage the inner surface 314 of the hole 304 formed in the glenoid cavity 104 as the force F is applied, and further secure the glenoid prosthesis 10 to the glenoid cavity 104.

Additionally, with reference now to Figures 11A and 11B, the alternative peg system 22" or 26" may comprise a primary peg 400. Typically, the primary peg 400 has a diameter D8 which is larger than the diameter D7 of the secondary pegs 302. The primary peg 400 may include at least one expandable fin 402, or a plurality of expandable fins 402. The expandable fins 402 may be generally comprised of a polymeric material such as polyethylene, or a bio-compatible metallic material, while the primary peg 400 may be formed of any suitable bio-compatible metallic material such as titanium, polymeric material, ceramic, or cobalt chromium.

The use of the alternative peg system 22' facilitates additional ingrowth of tissue and bone onto the primary peg 300, enabling for a more secure attachment of the glenoid prosthesis 10 to the glenoid cavity 104. In addition, the primary peg 300, may be coated with additional bone ingrowth materials, such as plasma, hydroxyapatite, calcium phosphate, or combinations thereof, to further increase the ingrowth of tissue and bone. Additionally, the use of a metallic uncemented primary peg 300 increases the stability of the glenoid implant while reducing the complexity of the glenoid implant surgery.

According to various embodiments, an alternative glenoid prosthesis 10' may be coupled to the glenoid cavity 104 by at least one sleeve 502, as shown in Figures 12A-14B. The glenoid prosthesis 10' may be similar to those described previously, but will generally include a first surface 504, second surface 506, and a peg system 508. The glenoid prosthesis 10' may be composed of any suitable polymeric material, such as ultra-high molecular weight polyethylene, polyether ether ketone or the like. The first surface 504 provides a surface for the humerus (not shown) to rotate against, while the second surface 506 is configured to correspond to the glenoid cavity 104. The peg system 508 generally couples the glenoid prosthesis 10' to the glenoid cavity 104.

The peg system 508 includes at least one peg 510, but generally may include a plurality of pegs 510. The pegs 510 may be composed of any suitable polymeric material, such as ultra-high molecular weight polyethylene, polyether ether ketone or the like. The pegs 510 include a first end 512 and a second end 514. The first end 512 of the pegs 510 generally serve to couple the pegs 510 to the second surface 506 of the glenoid prosthesis 10'. The first end 512 of the pegs 510 can be coupled to the second surface 506 of the glenoid prosthesis 10' via any appropriate method, such as adhesives, mechanical fasteners or may be formed onto the second surface 506 of the glenoid prosthesis 10' as a single part.

The second end 514 of each of the pegs 510 includes at least two forked portions 516 formed by a slit 518, however, additional forked portions 516 could be employed, and similarly, the second end 514 could be solid. The slit 518 generally extends to a mid-section 520 of the peg 510, and typically separates the second end 514 of the peg 510 in half. At the end of the forked portions 516 is an annular flange 522. The annular flange 522 facilitates the engagement of the peg 510 with the sleeve 502.

The sleeve 502 couples the glenoid prosthesis 10' to the glenoid cavity 104, as will be described in greater detail below. Generally, the number of sleeves 502 corresponds to the number of pegs 510. The sleeves 502 may be composed of any suitable bio-compatible metallic material, such as titanium, however, other materials could be employed such as cobalt chrome, ceramic, or biocompatible polymers. Typically, each of the sleeves 502 are of a diameter D9 which is smaller than a diameter D10 defined by the annular flange 522, but greater than a diameter D11 of the peg 510. The length L of the sleeves 502 may be any appropriate pre-selected length, however, the sleeve 502 is generally between 5 to 20mm in length.

In order to couple the glenoid prosthesis 10' to the anatomy 98, after cutting the incision 100, the surface of the glenoid cavity 104 may be reamed as shown in Figures 3A and 3B and discussed previously. Next, with reference to Figures 14A and 14B, a corresponding number of holes 524 can be drilled into the glenoid cavity 104 (not specifically shown). The diameter D12 of the hole 524 is generally slightly smaller than the diameter D13 of the sleeve 502 to provide for a press fit between the sleeve 502 and the hole 524. The number of holes 524 drilled into the surface of the glenoid cavity generally corresponds with the number of pegs 510 and sleeves 502 necessary to secure the glenoid prosthesis 10' into the glenoid cavity 104.

Next, the sleeves 502 may be inserted into the holes 524 formed in the glenoid cavity 104. The sleeves 502 may be inserted through any appropriate technique. For example, a rod (not shown) could be employed to rotate the sleeves 502 into the holes 524. In this regard, the sleeve can have a thread exterior surface, which is threadably coupled to the hole. Generally, the sleeves 502 will be inserted into the holes 524 to just below a surface 526 of the glenoid cavity 104, but the sleeves 502 could be inserted as far as desired into the holes 524.

After the insertion of the sleeves 502 into the holes 524, the force F may be applied to the second surface 506 of the glenoid prosthesis 10'. As the force F is applied, the pegs 510 will enter the holes 524 and due to the diameter D9 of the sleeves 502, the forked portions 516 of each of the pegs 510 are compressed as the annular flange 522 passes through the sleeve 502. Once the annular flange 522 exits the sleeve 502, the forked portions 516 re-expand, and the glenoid prosthesis 10' becomes coupled to the glenoid cavity 104.

The glenoid prosthesis 10' generally enables the surgeon to easily remove the glenoid prosthesis 10' if necessary as the surgeon can simply unsnap the glenoid prosthesis 10' from the sleeves 502. In addition, the glenoid prosthesis 10' may enable the surgeon to couple the glenoid prosthesis 10' to the glenoid cavity 104 arthroscopically. Further, the sleeves 502 may alternatively be threaded (not shown) and the pegs 510 may also include mating threads (not shown) to enable the pegs 510 to be screwed into the sleeves 502. Additionally, the sleeves 502 may be coated with materials such as plasma, hydroxyapatite, calcium phosphate or the like to facilitate increased bone and tissue growth.

The glenoid prosthesis 10, 10' according to various embodiments provides various surgical and biological benefits depending upon the selected embodiment. Not only may the glenoid prosthesis 10, 10' reduce surgical complexity and time, but it can further increase bone and tissue growth within the glenoid cavity 104. Additionally, any appropriate surface of the glenoid prosthesis 10, 10' may be coated with materials such as plasma, hydroxyapatite, calcium phosphate or the like to facilitate increased bone and tissue growth.

The description of the invention is merely exemplary in nature and, thus, variations that do not depart from the gist of the invention are intended to be within the scope of the invention. Such variations are not to be regarded as a departure from the spirit and scope of the invention.

## Claims

1. A modular prosthesis for replacing a portion of the anatomy comprising:
a first member defining a cavity, the cavity including a wall;
a second member defining a projection operable to interconnect with the cavity of the first member;
wherein at least one of the wall or the projection is resiliently deformable; and
wherein at least one of the first member or second member is fixed to the anatomy.

2. The modular prosthesis of Claim 1 wherein the first member further comprises:
a body defining a first surface;
at least one peg coupled to the first surface of the body;
wherein the peg defines the cavity.

3. The modular prosthesis of Claim 2 wherein the peg further comprises:
at least one annular protrusion disposed on an exterior surface of the wall;
at least one tip defining at least one curved recess to enable the projection to enter the cavity.

4. The modular prosthesis of Claim 1 wherein the cavity further comprises at least one spherical bore.

5. The modular prosthesis of Claim 4 wherein the projection further comprises at least one spherical protrusion operable to engage the spherical bore of the cavity.

6. The modular prosthesis of Claim 1 wherein the second member further defines a locking member operable to affix the second member to the anatomy.

7. The modular prosthesis of Claim 1. wherein the locking member includes at least one barb.

8. The modular prosthesis of Claim 1 further comprising:
at least one keel coupled to the first member; and
wherein the at least one keel is operable to engage the anatomy.

9. The modular prosthesis of Claim 2 wherein the modular prosthesis forms a glenoid prosthesis and the body further defines an articulated bearing surface.

10. A modular prosthesis for replacing a portion of the anatomy comprising:
a first member including at least one projection and at least one anchor, the at least one projection and at least one anchor operable to engage the anatomy;
wherein the projection is comprised of a metallic material; and
wherein the anchor is fixedly coupled to the anatomy.

11. The modular prosthesis of Claim 10 wherein the anatomy defines a plurality of openings operable to engage the at least one projection and at least one anchor.

12. The modular prosthesis of Claim 11 wherein the at least one anchor is retained in at least one of the plurality of openings by a material selected from the group comprising a resorbable material.

13. The modular prosthesis of Claim 10 wherein the at least one projection further comprises a coating selected from the group comprising a porous metal matrix, plasma spray, hydroxyapatite, calcium phosphate, or combinations thereof.

14. The modular prosthesis of Claim 10 wherein the at least one projection includes a first end adjacent to the first member and a second end, the at least one projection decreasing in diameter from the first end to the second end.

15. The modular prosthesis of Claim 10 wherein the at least one projection includes, at least one feature selected from the group comprising: an annular groove, a broached tooth, a roughened surface, or combinations thereof.

16. A modular prosthesis for replacing a portion of the anatomy comprising:
a first member including at least one resiliently deformable projection;
a second member defining an aperture operable to interconnect with the at least one resiliently deformable projection of the first member; and
wherein at least one of the first member or second member is fixed to the anatomy.

17. The modular prosthesis of Claim 16 wherein the second member further comprises at least one sleeve, the sleeve defining the aperture for receipt of the at least one resiliently deformable member, the at least one sleeve configured to engage the anatomy.

18. The modular prosthesis of Claim 17 wherein the at least one sleeve further comprises a biocompatible coating selected from the group comprising a porous metal matrix, porous plasma spray, hydroxyapatite, calcium phosphate, resorbable polymers, or combinations thereof.

19. The modular prosthesis of Claim 18 wherein the at least one sleeve is composed of a metallic material selected from the group comprising titanium, cobalt, chrome, ceramic, and combinations thereof.

20. The modular prosthesis of Claim 17 wherein the at least one resiliently deformable projection includes an annular flange adapted to interconnect with the at least one sleeve.

21. The modular prosthesis of Claim 17 wherein the sleeve comprises a threaded exterior surface.

22. The modular prosthesis according to Claim 17 wherein the sleeve is press-fit into an aperture formed in a bone.

23. The modular prosthesis according to Claim 17 further comprising bone cement disposed between the sleeve and the deformable member.

24. A method for replacing a portion of the anatomy comprising:
drilling at least one hole into the anatomy;
providing a first member including at least one cavity;
providing at least one second member operable to engage the anatomy;
engaging the at least one second member with the anatomy; and
interconnecting the at least one cavity of the first member with the at least one second member to couple the first member to the anatomy.

25. The method of Claim 24 further comprising:
forming at least one projection on the first member;
forming the at least one cavity in the projection of the first member;
providing at least one protrusion on the at least one second member;
applying a force to the first member to cause the at least one cavity of the first member to engage the at least protrusion of the second member to lock the first member to the second member.

26. The method of Claim 25 further comprising:
resiliently deforming the at least one cavity of the first member with the at least one protrusion of the second member.

27. The method of Claim 24 wherein the method of engaging the at least one second member with the anatomy further comprises:
attaching the at least one second member into the at least one hole formed in the anatomy.

28. A method for replacing a portion of the anatomy comprising:
drilling a first hole into the anatomy;
drilling a second hole into the anatomy;
providing a first member including a projection and at least one protrusion;
inserting the projection from the first member into the first hole; and
cementing the protrusion from the first member into the second hole to couple the first member to the anatomy.

29. The method of Claim 28 further comprising;
forming a taper on the projection prior of insertion of the projection into the first hole.

30. The method of Claim 28 wherein the projection is comprised of a metallic material and the protrusion is comprised of a polymeric material.

31. The method of Claim 28 further comprising:
forming at least one feature on the projection prior to insertion of the projection in the first hole; and
wherein the at least one feature is selected from the group comprising: an annular groove, a broached tooth, or combinations thereof.

32. The method of Claim 28 further comprising:
applying a coating to the projection, the coating selected from the group comprising porous metal matrix, plasma spray, hydroxyapatite, calcium phosphate, or combinations thereof.
